Europäisches Patentamt

European Patent Office

Office européen des brevets

(11) Publication number: **0 440 044 A1**

## EUROPEAN PATENT APPLICATION

(21) Application number: **91100510.6**

(22) Date of filing: **17.01.91**

(51) Int. Cl.⁵: **G01N 33/53, G01N 33/543**

(30) Priority: **31.01.90 US 472692**

(43) Date of publication of application:
**07.08.91 Bulletin 91/32**

(84) Designated Contracting States:
**DE ES FR IT**

(71) Applicant: **ABBOTT LABORATORIES**
**CHAD-0377, AP6D/2, One Abbott Park Road**
**Abbott Park, Illinois 60064-3500(US)**

(72) Inventor: **Kinders, Robert J.**
**31 N. Prindle Avenue**
**Arlington Heights, Illinois 60004(US)**
Inventor: **Daufeldt, Judith A.**
**17475 W. Dartmoor Drive**
**Grayslake, Illinois 60030(US)**
Inventor: **Hass, Michael G.**
**563 Sedgwick Drive**
**Libertyville, Illinois 60048(US)**
Inventor: **Henslee, Jerry G.**
**1309 Bull Creek Drive**
**Libertyville, Illinois 60048(US)**
Inventor: **Ostrow, David H.**
**985 Manchester Court**
**Lake Zurich, Illinois 60047(US)**

(74) Representative: **Modiano, Guido et al**
**MODIANO, JOSIF, PISANTY & STAUB**
**Modiano & Associati Via Meravigli, 16**
**I-20123 Milano(IT)**

(54) Avoidance of human anti-mouse antibody interference in in vitro diagnostic testing.

(57) The present invention enables the performance of immunoassays which use mouse monoclonal antibody assay reagents but in which human anti-mouse antibody-induced interference is substantially avoided without the need for cumbersome sample pretreatment steps and without denaturing the analyte epitope(s) necessary to the binding reactions of the immunoassay. The present invention also enables the determination of the presence or amount of interfering antibodies in the test sample. For example, bridging assays for the detection of the presence or amount of human anti-mouse antibodies in a test sample can be configured to determine anti-allotype, anti-idiotype antibodies and/or anti-isotype antibodies. Homogeneous and heterogenous assay methods and sandwich and competitive assay formats are described.

HAMA BRIDGING OR COMPETITION

SOLID PHASE ANTIBODY  HUMAN ANTI-MOUSE ANTIBODY  ENZYME-LABELED ANTIBODY FRAGMENT

FIG. 1

AVOIDANCE OF HUMAN ANTI-MOUSE ANTIBODY INTERFERENCE IN *IN VITRO* DIAGNOSTIC TESTING

**BACKGROUND OF THE INVENTION**

1. Field of the Invention

The present invention relates to the field of immunoassay methods. More particularly, the invention relates to the detection of antigens in body fluids, while substantially eliminating the assay interference caused by the presence of human anti-mouse antibodies in these fluids.

2. Description of Related Art

Monoclonal antibodies are often administered to humans for various *in vivo* diagnostic and therapeutic procedures. For example, because monoclonal antibodies are extremely specific to the antigens against which they are produced, they can be used to specifically direct cytotoxic substances or imaging agents to tumor sites in the body. Several current cancer therapies use mouse monoclonal antibodies, which were raised against human tumor-associated antigens, as therapeutic agents to carry cytotoxins to a tumor site. In addition, radiolabeled mouse monoclonal antibodies, which are specific for a tumor-associated antigen, may be injected into patients to accurately determine the sites of extremely small tumors. Other mouse monoclonal antibodies, such as antibodies to lymphocyte markers, to proteins associated with tissue damage (such as creatine kinase), and to endotoxins, have been injected into humans to study therapeutic or diagnostic procedures. The immunoglobulin $IgG_1$ is the predominant mouse monoclonal antibody isotype being used in these procedures. Many patients produce human anti-mouse antibodies in response to the injection of mouse antibodies during these procedures. The presence of circulating human anti-mouse antibodies in such patients can complicate the subsequent *in vivo* administration of therapeutic and diagnostic agents containing mouse monoclonal antibody by interfering with the intended functional activity of that antibody. Furthermore, the presence of human anti-mouse antibodies has been found to interfere with the results of *in vitro* diagnostic assays which use mouse monoclonal antibodies as assay reagents. (Traub, et al., Cancer Res. 48:4002-4006, 1988; Moseley, et al., J. Immunol. Meth. 106:1-6, 1988; Jaffers, et al., Transplantation 41:572-578, 1986; and Herlyn, et al., J. Immunol. Meth. 85:27-38, 1985.)

The *in vitro* quantification of tumor markers in blood, such as alpha-fetoprotein (AFP), human chorionic gonadotropin (HCG) and carcinoembryonic antigen (CEA), is important for monitoring disease activity in cancer patients. A number of procedures have been suggested in the literature to reduce the interference caused by human anti-mouse antibodies in immunoassays which measure or detect these tumor-associated antigens and which use mouse monoclonal antibody assay reagents. One method, used to reduce human anti-mouse antibodies interference in immunoassays for CEA, involves pretreating the test sample by heating the sample at 70 to 90° C for approximately 15 minutes. The interfering protein is thereby precipitated to provide a supernatant containing CEA. Although this method is useful for CEA immunoassays, it is not suitable in other immunoassays in which the analyte (e.g., antigen) has a heat-sensitive epitope. (Primus, et al., Clin. Chem. 34:261-264, 1988.) Another test sample pretreatment method involves passing the CEA test sample through an immunoadsorbent column containing Protein A, Protein G or anti-human IgG antibody to which the human anti-mouse antibodies bind (Primus, et al. Clin. Chem. 34:261-264, 1988). Column chromatographic methods, however, are very cumbersome and are not recommended for the routine handling of patient samples.

A slightly more convenient pretreatment method involves the addition of polyethylene glycol to the plasma sample, followed by vortex mixing, incubation and centrifugation, to precipitate the human anti-mouse antibodies from the CEA test sample (Primus, et al., Clin. Chem. 34:261-264, 1988.) This method, however, is still too cumbersome to be performed as part of a standard assay procedure in a clinical laboratory. Other methods for reducing human anti-mouse antibodies interference in immunoassays involve the use of perchloric acid extraction (Kim, et al., U.S. Patent Number 4,180,556), or the addition of excess liquid mouse polyclonal or monoclonal antibody (Hansen, et al., Clin. Chem. 35:146-151, 1989; Newman, et al., Clin. Chem. 35:1743-1746, 1989) to bind human anti-mouse antibodies and thereby decrease human anti-mouse antibodies reactivity with the assay reagents.

The conventional pretreatment methods, however, add steps to the assay protocol and/or are inefficient in removing human anti-mouse antibodies. Therefore, there is a need for a simple method to substantially avoid any human anti-mouse antibodies interference in immunoassays which use mouse monoclonal antibodies, and that can be performed without cumbersome sample pretreatment steps and without denaturing the analyte epitope(s) necessary to the binding reactions of the immunoassay.

## SUMMARY OF THE INVENTION

The present invention includes novel immunoassay methods and kits for the detection of analytes of interest while substantially avoiding the interference effects of human anti-mouse antibodies, thereby enabling the accurate monitoring of circulating antigen levels. The avoidance of interference is meant to include the elimination of human anti-mouse antibodies from the immunoassay system or at least the reduction of such interference effects to a level wherein the unwanted antibodies do not significantly affect the assay results.

In one immunoassay method for detecting the presence or amount of an analyte of interest in a patient sample, wherein the sample contains human anti-mouse antibodies produced by the prior immunization of the patient with mouse antibodies, the sample is combined with a capture antibody to form a mixture. The capture antibody is selected to be xenogeneic in relation to the immunizing mouse antibodies and capable of binding the analyte of interest. The analyte and capture antibody thereby form a complex which can be separated from the mixture and reacted with an indicator reagent. The indicator reagent typically includes a detectable label attached to an antibody, wherein the antibody binds to the analyte/capture antibody complex thereby forming a detectable complex in relation to the presence or amount of analyte in the test sample. The method can also include the addition of mouse IgG to the analyte, the capture antibody or the test mixture, wherein the IgG decreases the crossreactivity of the capture antibody with the human anti-mouse antibodies.

If the analyte/capture antibody complex is associated with a solid phase, the complex can be separated from the mixture or test system by washing said solid phase or by other separation techniques well-known in the art such as precipitation, electrophoresis or agglutination techniques. The assay method can be performed as a one-step or two-step assay format, and the method can be performed as a sandwich or competitive assay. In addition, the principles of the present invention are applicable to many assay formats to enable the user to avoid the interfering effects of antibodies which occur in test samples due the the immunization of the test subject with antibodies from another species, and therefore, the present invention is not limited to assay interference induced by human anti-mouse antibodies. The assays can be performed in either one-step or two-step assay configurations.

In another aspect of the present invention, the presence or amount of interfering antibodies in the test sample can be determined. For example, bridging assays for the detection of the presence or amount of human anti-mouse antibodies in a test sample can be configured to determine anti-allotype, anti-idiotype antibodies and/or anti-isotype antibodies. The assay can be performed by contacting the test sample with a mouse monoclonal antibody to form a test mixture, wherein the human anti-mouse antibodies bind to an isotypic epitope on the mouse monoclonal antibody. The mouse monoclonal antibody is typically an IgG or IgM antibody. A mouse monoclonal antibody/human anti-mouse antibody complex is formed, and the resultant complex can be separated from the test mixture. A detection means is then used to determine the presence or amount of the complex, thereby detecting the presence of anti-allotype and anti-isotype antibodies. The detection means typically involves an indicator reagent including a detectable label attached to an immunoreactant. The immunoreactant binds to the mouse monoclonal antibody or the human anti-mouse antibodies, e.g., a labeled anti-human antibody.

A bridging assay may also be configured such that the test sample is combined with a mouse monoclonal antibody to form a test mixture, wherein the mouse monoclonal antibody is the same as the immunizing antibody, and wherein the human anti-mouse antibodies bind to that mouse monoclonal antibody to form a mouse monoclonal antibody/human anti-mouse antibody complex. The complex can be detected as described above and in the specific examples which follow to determine both anti-idiotype and anti-isotype antibodies. For example, the indicator reagent can include a detectable label attached to an immunoreactant, wherein the immunoreactant is an anti-human antibody, the immunizing mouse antibody or a fragment of the immunizing mouse antibody.

A bridging assay for the determination of the presence or amount of anti-idiotype human anti-mouse antibodies in a test sample is also provided wherein the the presence or amount of anti-isotype antibodies in the test sample is determined, the presence or amount of anti-idiotype and anti-isotype antibodies in the test sample is determined and the assay results are correlated to determine the presence or amount of anti-idiotype antibodies. Alternatively, the capture binding member and specific binding member of the indicator reagent can be fragments of the immunizing antibody which bind the idiotypic epitope but little or no isotypic epitope of the interfering antibody.

The various assays described can also include the use of at least one ancillary specific binding member to complete the detectable complex. Test kits for performing any of these assay methods can also be produced. A complete test kit will include the appropriate capture antibody and an appropriate indicator reagent, and can optionally include a sample diluent containing mouse serum.

## BRIEF DESCRIPTION OF THE DRAWINGS

Figure 1 illustrates the possible false positive [Fig. 1(a), (b) or (c)] and false negative [Fig. 1 (d) or (e)] assay results which can result from the presence of human anti-mouse antibodies in a test sample whether or not the analyte of interest is present in the test sample.

Figure 2 illustrates the importance of epitope specificity in the construction of an assay to measure analyte of interest. Even though a large number of antibodies may react specifically with any antigen, the combination of any two of such antibodies may not enable the production of a sandwich assay for that antigen.

Figure 3 illustrates the measurement and characterization of human anti-mouse antibodies.

Figure 4 illustrates the results of a human anti-mouse antibody bridging assay that was designed and used to measure and characterize the immune response of a patient who had been injected with mouse monoclonal antibody C110.

Figure 5 illustrates the use of antibody fragments in an assay for human anti-mouse antibodies, to elucidate the specificity of human anti-mouse antibodies.

Figure 6 illustrates the response of human anti-mouse antibodies to an antibody that recognizes an epitope found in multiple copies on a single antigen molecule.

## DETAILED DESCRIPTION OF THE INVENTION

Mouse monoclonal antibodies, which are used in *in vivo* therapeutic and diagnostic agents, are raised in mice by eliciting a specific immune response to the antigen of interest. For example, mouse anti-CEA monoclonal antibodies are raised in response to and are capable of recognizing a specific antigenic determinant or epitope of the CEA molecule, i.e., that part of the CEA molecule to which the antibody directly binds. Binding occurs because the resultant antibody includes a binding site that is complementary to the antigenic determinant. Upon injection of such an antibody into a patient for diagnostic or therapeutic purposes, the mouse monoclonal antibody can have the side-effect of eliciting an immune response which results in the patient's production of human anti-mouse antibodies. As a consequence, the mouse monoclonal antibody that is complementary to a specific antigen can also act as an antigen to elicit the production of human anti-mouse antibodies with which the mouse monoclonal antibody can specifically bind.

The basis of an IgG antibody's specificity resides at one end of its four constituent amino acid chains. Four such chains make up each antibody molecule: two identical heavy or long chains, partially flanked by two identical light chains or short amino acid segments which form the two "arms" of the antibody molecule. Much of each chain includes a "constant region" whose amino acid sequence is similar for every chain within a broad class of antibodies raised in a given animal. Each chain also includes a "variable region" that has a unique amino acid chemistry and conformation for each antibody clone raised against a given antigen. At the end of each of the antibody's two arms, the variable regions of a heavy chain and a light chain form a unique binding site which enables the antibody to interact with its specific antigenic determinant. Assay interference from human anti-mouse antibodies arises because an antibody can in turn become the target of other antibodies which recognize the primary antibody's unique molecular characteristics. This recognition results in antibody-antibody reactions known as idiotype - anti-idiotype reactions, wherein the primary antibody acts as an antigen and elicits a specific immune response.

Antibodies which are elicited in response to the presence of a primary antibody can be classified into one of two main groups: anti-isotype antibodies and anti-idiotype antibodies. Anti-isotype antibodies recognize antigenic determinants in the constant region of the primary antibody's heavy and light chains, i.e., that region whose amino acid sequence is substantially similar for every antibody produced by a mouse. Thus, anti-isotype antibodies can bind to many different antibodies produced by a given animal species. Conversely, anti-idiotype antibodies recognize the primary antibody's unique antigenic determinants, i.e., that region of the primary antibody which was elicited specifically in response to a given antigen. Thus, anti-idiotype antibodies will bind only to certain primary antibodies.

In the present invention, it has been determined that a single injection of as little as one milligram of mouse monoclonal antibody is sufficient to generate a human anti-mouse antibody response. Such an injection of a mouse monoclonal antibody can result in the patient's production of both anti-isotype and anti-idiotype IgG class human anti-mouse antibodies. Observation of the time course of the response has shown that the anti-idiotype antibodies appear later, but remain in circulation longer. It has also been determined

5

that the presence of human anti-mouse antibodies can cause false negative or false positive test results in immunoassays including sandwich-type immunoassays designed to detect tumor-associated antigens.

For example, in a conventional CEA sandwich immunoassay, a first mouse anti-CEA monoclonal antibody is immobilized upon a solid support. A test sample suspected of containing CEA (antigen) is contacted to the solid support, and the antigen and antibody bind, thereby immobilizing the antigen upon the support. The support and sample are also contacted with a second mouse anti-CEA monoclonal antibody which is conjugated to a detectable label. The second antibody binds to a separate determinant on the CEA molecule, thereby labeling any antigen that is present on the support. Typically, the assay procedure is performed as a single step wherein the test sample, the immobilized solid phase antibody and the labeled antibody are simultaneously combined and incubated.

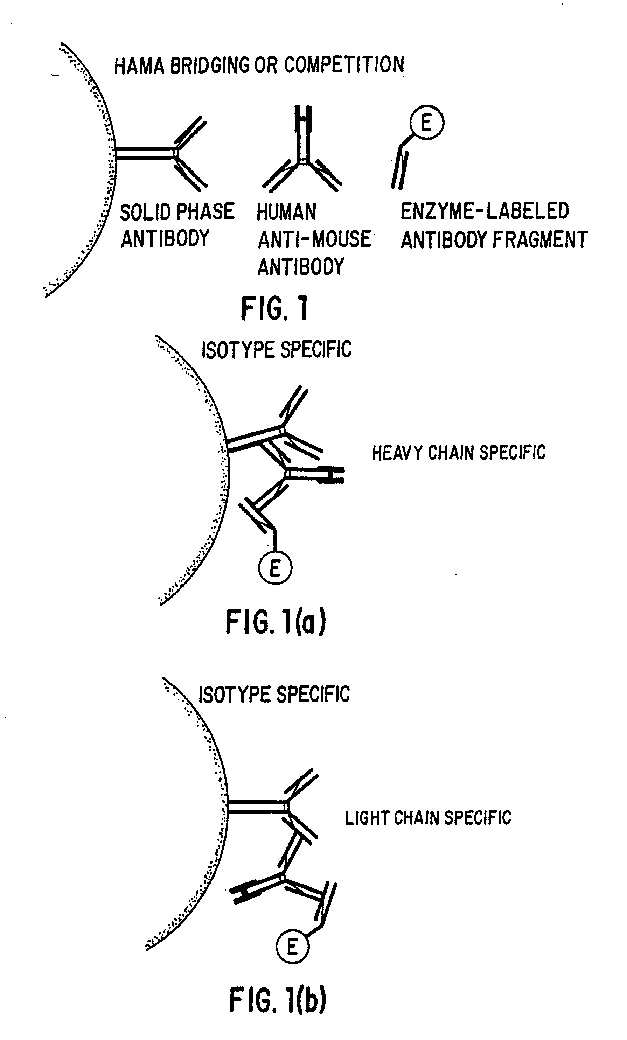

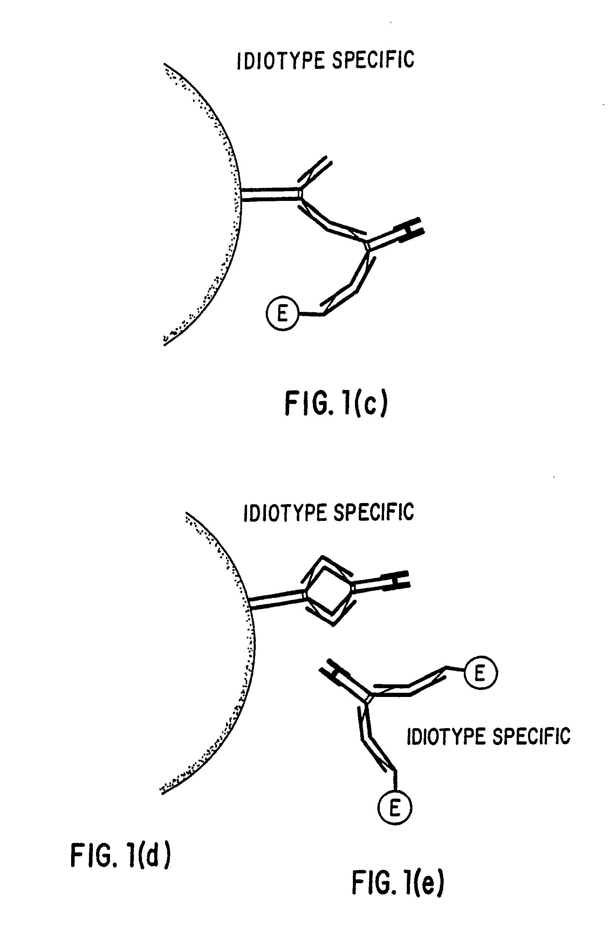

Human anti-mouse antibodies can be present in a test sample due to a patient's prior immunization to mouse antigen, through either a passive immunization via allergic reaction or an active immunization via the injection of mouse antibodies. Anti-isotype human anti-mouse antibodies, i.e., antibodies that are specific for the constant region of the mouse monoclonal antibodies used as assay reagents, can result in the "bridging" of solid phase and labeled mouse antibodies used as assay reagents. The bridging reaction is illustrated in Figures 1(a) and 1(b) wherein the human anti-mouse antibody is shown to bind to both a solid phase mouse monoclonal anti-analyte antibody and a labeled mouse anti-analyte antibody fragment. Thus, even in the absence of the analyte which was to be detected, the consequence of such human anti-mouse antibody interference with the immunoassay is the immobilization of labeled antibody upon the solid phase, thereby causing false positive assay results.

Anti-idiotype human anti-mouse antibodies are antibodies present in the test sample due to the prior injection of the patient with a mouse monoclonal antibody. These anti-idiotype antibodies can be specific for the variable region of the mouse monoclonal antibody used as an assay reagent when the identical mouse monoclonal antibody also served as an immunogen during the therapeutic or diagnostic *in vivo* administration of that antibody to the patient. Thus, in an assay where the same mouse monoclonal antibody is used as the solid phase antibody and as the labeled antibody. anti-idiotype human anti-mouse antibodies can bridge the solid support and labeled antibodies, again resulting in false positive assay results. Such anti-idiotype antibody bridging is illustrated in Figure 1(c) wherein the human anti-mouse antibody is shown to bind the variable (idiotype) region of both the solid phase antibody and the labeled antibody fragment. Alternatively, anti-idiotype human anti-mouse antibodies can directly compete with the test sample antigen for the available binding sites on the solid support antibody and/or labeled antibody assay reagents. Such competitive binding of human anti-mouse antibodies is illustrated in Figure 1(d) and (e) wherein the human anti-mouse antibody would prevent analyte in the test sample from binding to the assay reagents. The competitive human anti-mouse antibody binding can result in the "blocking" of the solid phase and labeled antibodies due to human anti-mouse antibodies occupying the available assay reagent binding sites. Thus, human anti-mouse antibodies can occupy the binding sites on the assay reagents such that the analyte cannot bind the assay reagents, thereby resulting in false negative assay results. In a homologous assay, wherein the capture binding member and the specific binding member component of the indicator reagent are antibodies or antibody fragments having the same idiotype, then the results shown in (c), (d) or (e), or a combination thereof, can occur. In an assay, wherein the immunizing agent antibody or a fragment thereof is used as either the capture binding member or the specific binding member component of the indicator reagent, then the results shown in (d) or (e) can occur.

Once the present investigators had determined the mechanisms of human anti-mouse antibody interference, they designed the immunoassay methods of the present invention which advantageously avoid human anti-mouse antibody interference and do not require complicated test sample pretreatment procedures. The avoidance of significant human anti-mouse antibody interference includes the capability to detect the desired analyte even though human anti-mouse antibodies are present in the test sample.

It will be appreciated by those skilled-in-the-art that the concepts of the present invention are applicable to any immunoassay formats, e.g., competitive assays, sandwich assays, homogeneous and heterogenous assays, which are susceptible to interference caused by the presence of extraneous antibodies in the test sample which will specifically bind those antibodies which are used as assay reagents. Human anti-mouse antibodies are used in the description of the present invention only as an example of such an interfering antibody which has an increasing frequency of occurrence due to the widespread use of mouse antibodies in therapeutic and diagnostic agents. Interfering antibodies can also arise due to immunization with rabbit antibodies, goat antibodies, etc. Thus, the presence of interfering antibodies elicited against any given animal's immunoglobulin can result in false immunoassay values if antibodies from the same species are also used as an assay reagent. The present invention, however, enables the detection of the target analyte in a test sample which contains an interfering antibody. A xenogeneic antibody is advantageously used as the capture antibody in this assay format. The term "xenogeneic" is used to denote an antibody from a species different from the species against which the interfering antibody was produced. For example, a polyclonal goat anti-CEA antibody is a xenogeneic antibody in relation to the monoclonal mouse antibody which elicited the production of human anti-mouse antibodies present in the patient's test sample.

The present invention also enables assays to avoid interference which may be caused by allotype reactivity. Allotypic markers or allotypes are epitopes that are genetic variants of molecules (in this instance, immunoglobulin molecules) found on the immunoglobulins of some but not all members of a species. For example, mouse monoclonal antibodies made from fusions of spleen cells from Balb/C mice have different allotypes than antibodies made from fusions of spleen cells from non-Balb/C mice. Thus, human anti-mouse antibodies that are produced in response to antibodies from one strain of mice may have different allotypic epitopes from human anti-mouse antibodies that are produced in response to antibodies from a different strain of mice. These different allotypic epitopes are not related to antibody specificity.

The present invention also includes the detection of interfering antibodies in a test sample which contains a target analyte. It has been found that a bridging assay format can be used for such detection, wherein the capture antibody and labeled antibody are chosen based upon the interfering antibody's ability to simultaneously bind the assay reagent antibodies and upon the assay reagent antibodies' inability to simultaneously bind the target analyte.

Before proceeding further with the description of the various specific embodiments of the present invention, a number of terms will be defined.

The term "analyte" refers to either the the interfering antibody, such as human anti-mouse antibody, to be detected or an antigen of interest, including but not limited to the tumor-associated antigens.

The term "test sample" typically refers to body fluid samples such as plasma, serum, ascites, lymphatic fluids, cerebral spinal fluid, nipple fluid discharge, urine and other body fluids that may contain the analyte of interest. Optionally, the test sample can be diluted in a suitable diluent buffer, such as phosphate buffered saline with serum components, to provide a sample volume that is required by the particular immunoassay.

The term "specific binding member" refers to a member of a specific binding pair, i.e., two different molecules wherein one of the molecules through chemical or physical means specifically binds to the second molecule. In addition to antigen and antibody specific binding pairs, other specific binding pairs include, biotin and avidin, carbohydrates and lectins, complementary nucleotide sequences, complementary peptide sequences, effector and receptor molecules, enzyme cofactors and enzymes, enzyme inhibitors and enzymes, a peptide sequence and an antibody specific for the sequence or the entire protein, polymeric acids and bases, dyes and protein binders, peptides and specific protein binders (e.g., ribonuclease, S-peptide and ribonuclease S-protein), and the like. Furthermore, specific binding pairs can include members that are analogs of the original specific binding member, for example an analyte-analog. If the specific binding member is an immunoreactant it can be, for example, an antibody, antigen, hapten, or complex thereof. If an antibody is used, it can be a monoclonal or polyclonal antibody, a recombinant protein or antibody, a mixture(s) or fragment(s) thereof, as well as a mixture of an antibody and other specific binding members. The details of the preparation of such antibodies and their suitability for use as specific binding members are well-known to those skilled-in-the-art.

The term "indicator reagent" refers to an assay reagent comprising a detectable label directly or indirectly attached to a specific binding member which is capable of directly or indirectly binding to the analyte and thereby indicate the presence, absence or amount of the analyte in a test sample. A variety of different indicator reagents can be formed by varying either the label or the specific binding member. In general, the indicator reagent is detected after it has formed a complex with either the analyte or a complementary specific binding member, but the unbound indicator reagent can also be detected.

The term "label" refers to any substance which is attached to a specific binding member and which is capable of producing a signal that is detectable by visual or instrumental means. Suitable labels for use in the present invention can include chromogens; catalysts; fluorescent compounds; chemiluminescent compounds; radioactive isotopes; direct visual labels including colloidal metallic and non-metallic particles, dye particles, enzymes or substrates, or organic polymer latex particles; liposomes or other vesicles containing signal producing substances; and the like.

A large number of enzymes suitable for use as labels are disclosed in U.S Patent No. 4,275,149, columns 19-23, herein incorporated by reference. For example, an enzyme/substrate signal producing system useful in the present invention is the enzyme alkaline phosphatase wherein the substrate used is nitro blue tetrazolium-5 bromo-4-chloro-3-indolyl phosphate or a derivative or analog thereof. If horse-radish peroxidase is used, o-Phenylenediamine is added as an enzyme substrate to form a colored product which can be detected and/or measured visually or instrumentally.

7

In an alternative signal producing system, the label can be a fluorescent compound where no enzymatic manipulation of the label is required to produce a detectable signal. Fluorescent molecules such as fluorescein, phycobiliprotein, rhodamine and their derivatives and analogs are suitable for use as labels in this system.

An especially preferred class of labels includes the visually detectable, colored particles which enable a direct colored readout of the presence or concentration of the analyte in the test sample without the need for using additional signal producing reagents. Materials for use as such particles include colloidal metals, such as gold, and dye particles as disclosed in U.S. Patent Numbers 4,313,734 and 4,373,932. The preparation and use of non-metallic colloids, such as colloidal selenium particles, are disclosed in co-owned and copending U.S. Patent Application Serial No. 072,084, filed July 9, 1987, which is incorporated by reference herein. Organic polymer latex particles for use as labels are disclosed in co-owned and copending U.S. Patent Application Serial No. 248,858, filed September 23, 1988, which is incorporated by reference herein. The selection of a particular label is not critical, so long as the label is capable of generating a detectable signal either by itself or in conjunction with one or more additional signal producing substances.

The term "signal producing component" refers to any substance capable of reacting with another assay reagent or the analyte to produce a reaction product or signal that indicates the presence of the analyte and that is detectable by visual or instrumental means. "Signal production system", as used herein, refers to the group of assay reagents that are needed to produce the desired reaction product or signal. For example, one or more signal producing components can be used to react with a label and generate the detectable signal, i.e., when the label is an enzyme, amplification of the detectable signal is obtained by reacting the enzyme with one or more substrates or additional enzymes to produce a detectable reaction product.

The term "capture binding member" refers to a specific binding member which can bind directly or indirectly to the analyte or indicator reagent and which is bound or is capable of being bound to a solid phase, or is capable of being precipitated, such that the capture binding member can be separated from the test sample and other assay reagents.

The term "capture reagent" refers to a capture binding member which is directly or indirectly attached to a solid phase material to enable the separation of the capture binding member, and analyte or indicator reagent that is bound thereto, from unbound analyte and assay reagents. Typically, the attachment of the capture binding member to the solid phase material is substantially irreversible and can include covalent mechanisms. The capture reagent of the present invention, however, is not limited to a capture binding member bound to an insoluble solid phase material. In an agglutination assay, the capture binding member of the capture reagent can be bound to a soluble carrier material such as bovine serum albumin.

In preforming a capture reagent to be used in an assay, once the capture binding member, e.g., analyte specific antibody, is immobilized upon the solid phase, the remaining surface area of the solid phase is generally blocked with a suitable protein solution, such as bovine serum albumin, to prevent non-specific binding of protein to the support. The solid support is then washed with an appropriate solution to remove any excess blocking solution and/or unbound capture binding member.

Once complex formation occurs between the assay components, the solid phase can be used as a separation mechanism. For example, the reaction mixture can be contacted with the solid phase material, and the newly formed reaction complex(es) are retained by the solid phase material. Alternative methods can be used to perform this separation step, such as using a solid phase which itself binds to the capture binding member; affixing to the solid phase a binding member that is specific for the capture binding member; or affixing to the solid phase a reactive agent, such as a charged substance, which will attract and bind an oppositely charged substance that has been bound to the capture binding member, as disclosed in co-owned and copending U.S. Patent Application Serial No. 150,278, filed January 29, 1988, which is incorporated by reference herein.

The assay device of the present invention can have many configurations, several of which are dependent upon the material chosen for the solid phase. The term "solid phase material" refers to any suitable chromatographic, bibulous, porous or capillary material or other conventional solid material, well-known to those skilled-in-the-art for use in immobilizing specific binding members. In the present invention, the solid phase material can include a fiberglass, cellulose or nylon pad for use in a flow-through assay device having one or more layers containing one or more of the assay reagents; a dipstick for a dip and read assay; a test strip for chromatographic (e.g., paper or glass fiber) or thin layer chromatographic (e.g., nitrocellulose) techniques in which one or all of the reagents are contained in separate zones of a single strip of solid phase material; or an absorbent material well known to those skilled in the art. The solid phase material can also include, without limitation, polyacrylamide beads, polystyrene beads or tubes, magnetic beads, a microtitre plate with one or more reaction wells or a glass or plastic test tube.

Natural, synthetic or naturally occurring materials that are synthetically modified, can be used as a solid phase material including polysaccharides, e.g., cellulose materials including paper, cellulose and cellulose derivatives such as cellulose acetate and nitrocellulose; silica; fiberglass; inorganic materials such as deactivated alumina, diatomaceous earth or other inorganic finely divided material uniformly dispersed in a porous polymer matrix, with polymers such as vinyl chloride, vinyl chloride-propylene copolymer, and vinyl chloride-vinyl acetate copolymer; cloth, both naturally occurring (e.g., cotton) and synthetic (e.g., nylon); porous gels such as silica gel, agarose, dextran and gelatin; polymeric films such as polyacrylamide; magnetic particles; microtitre plates; polystyrene tubes; protein binding membranes; agarose; Sephadex® - (Pharmacia Fine Chemicals, Inc., Piscataway, N.J.); Trisacryl® (Pointet-Girard, France); silicon particles; porous fibrous matrixes; and the like. The solid phase material should have a reasonable inherent strength or strength can be provided by means of a support, and it should not interfere with the production of a detectable signal.

Optionally, the specific binding member of the capture reagent can be affixed to particles, e.g., microparticles. These microparticles can serve as the solid phase material and be retained in a column, suspended in a mixture of soluble reagents and test sample, or retained and immobilized by another solid phase base material. By "retained and immobilized" is meant that the microparticles, associated with the solid phase base material, are not capable of substantial movement to positions elsewhere within that material. The microparticles can be selected by one skilled-in-the-art from any suitable type of particulate material including those composed of polystyrene, polymethylacrylate, polypropylene, polytetrafluoroethylene, polyacrylonitrile, polycarbonate or similar materials. The size of the microparticles is not critical, although it is preferred that the average diameter be smaller than the average pore size of the solid phase base material if such is used.

The term "ancillary specific binding member" refers to a specific binding member used in addition to the capture binding member and the indicator reagent which becomes a part of the detectable binding complex. One or more ancillary specific binding members can be used in an assay. For example, an ancillary specific binding member can be used in an assay where the capture binding member is capable of binding the ancillary specific binding member which is in turn capable of binding the solid phase.

It will be appreciated by those skilled-in-the-art that the selection of any given label, ancillary binding member or solid phase material is generally not critical to the present invention. The materials are chosen to optimize the results provided by the chosen assay configuration.

Analyte of interest assay

The analyte assay of the present invention is preferably performed as a two-step heterogeneous sandwich assay. While the sandwich immunoassay is a well-known immunoassay technique, the present invention provides a novel use for the known procedure, thereby enabling the avoidance of assay interference caused by unwanted antibodies, such as human anti-mouse antibodies, in the test sample.

In the assay, the test sample is first incubated with a capture reagent, e.g., an antibody adsorbed upon or otherwise attached to a solid phase, such that the analyte binds to the antibody. Following this incubation, the solid phase is washed to remove unreacted sample. The wash also removes interfering antibody from the test system while leaving the analyte immobilized upon the capture reagent. The immobilized analyte is then reacted with a labeled antibody (indicator reagent), thereby producing a detectable capture reagent/analyte/indicator reagent complex In one preferred embodiment, the capture reagent antibody is a xenogeneic antibody, specific for the analyte to be detected, directly or indirectly attached to or immobilized upon a solid phase. For example, in an assay designed to avoid interference caused by the presence of human anti-mouse antibodies in the test sample, a non-mouse antibody specific for the analyte would be used as the capture reagent; the probability that anti-mouse isotype or anti-mouse idiotype antibodies will be immobilized upon the solid phase is minimized because of the differences in the amino acid sequences in the immunoglobulins of mice versus those of other animals. Although either polyclonal or monoclonal antibodies can be used to form the capture reagent, a polyclonal antibody is most preferred. In a heterogeneous assay, the antibody component of the indicator reagent can be, but is not required to be, a xenogeneic antibody because the interfering antibody is typically removed from the test system by washing prior to the reaction of the analyte and indicator reagent. With this two-step assay protocol the interfering antibody is prevented from bridging the xenogeneic capture antibody and the labeled antibody. Thus, the assay is effective in determining the actual concentration of analyte in the test sample despite the presence of interfering antibody.

If a monoclonal antibody is used as the capture binding member, it is most preferably derived from a species different from that species which is most often used to produce the diagnostic or therapeutic antibodies that are injected into patients. Generally, mouse IgG$_1$ antibodies are used for *in vivo* human treatment or diagnosis of tumor-associated antigens. Therefore, in an *in vitro* assay for tumor-associated antigen, the monoclonal antibody attached to the solid phase would preferably be a non-mouse monoclonal antibody, or at least a mouse IgM antibody rather than a mouse IgG$_1$ antibody. Other preferred antibodies can be derived from rats, goats, sheep, rabbits, guinea pigs, horses, etc. by well-known methods.

While the two-step sandwich assay format enables the performance of assays for an analyte of interest while avoiding assay interference caused by extraneous antibodies, a one-step assay can also be performed. In the one-step assay, the capture binding member, test sample and indicator reagent are combined simultaneously or without an intervening wash step. If a one-step assay format is used then it is preferable that one or both binding members be xenogeneic relative to the immunizing antibody (e.g., a double polyclonal antibody assay) and that serum from the species from which the immunizing antibody was derived be added to the test system.

For most test samples, the above described two-step assay is sufficient to detect the analyte of interest while avoiding interference from human anti-mouse antibodies. Some test samples, however, may contain very high titers of such interfering antibodies. In these instances, it is preferable to also add mouse serum to the test system or assay reagents, such that the additional mouse antibodies will bind the interfering human anti-mouse antibodies and thereby decrease the reactivity of such antibodies with the assay reagents. The combination of the addition of mouse serum to the test system and the two-step assay format using a xenogeneic antibody as the capture antibody produces a preferred assay format.

The analyte assay can also be performed in a competitive assay format. For example, the analyte or analyte analog is immobilized upon a solid phase and competes with analyte in the test sample for binding to a labeled xenogeneic binding member. Alternatively, the xenogeneic binding member can be immobilized upon the solid phase and the analyte of interest and a labeled analyte or labeled analyte analog compete for binding.

Homogeneous assays can also be performed. In such assays however, human anti-mouse antibody interference is disrupted by the use of xenogeneic antibodies, as the separation of bound and unbound analyte is not preformed.

Human anti-mouse antibody assay

To demonstrate the presence of human anti-mouse antibodies in test samples, such as the test samples for the detection of tumor-associated antigen, it was first necessary to design an assay that would detect only human anti-mouse antibodies even though the target antigen was present. Preferably, the same antibody that was used for *in vivo* purposes and which led to the production of interfering antibodies is also used as the assay reagent antibody because it will provide maximum assay sensitivity in that it is the exact immunizing agent which gave rise to the interfering antibody. Thus, the same mouse monoclonal antibody that was used for *in vivo* therapeutic treatment of the patient, and which led to the production of human anti-mouse antibodies, is also used as the assay reagent antibody.

Specific examples of such mouse monoclonal antibodies are described by Hammarstrom, et al. (Cancer Research 49: 4852-4858, 1989, which is incorporated by reference herein) wherein the epitope reactivities of 52 well-characterized anti-CEA monoclonal antibodies from 11 different research groups are discussed. Further examples of well-characterized mouse monoclonal antibodies are provided in Table 1.

## TABLE 1

### EXAMPLES OF MOUSE MONOCLONAL REAGENT ANTIBODIES

| Monoclonal Antibody | Immunogen | Isotype | Characteristics |
|---|---|---|---|
| B72.3 | Liver metastasis of a primary human breast cancer | $IgG_1$ | Directed to 6-Sialosyl Tn Antigen; carbohydrate found on mucin glycoprotein (tumor associated glycoprotein [TAG-72] (Will not bind CEA) |
| C110 | CEA | $IgG_1$ | Directed to a proteinaceous epitope in region N of CEA |
| OC125 | Human ovarian cancer cell line (OvCa 433) | $IgG_1$ | Directed to a protein/carbohydrate epitope on mucin glycoprotein expressed in ovarian cancer |
| H8C2 | CEA | $IgG_1$ | Directed to a proteinaceous epitope in domain 1 of CEA |
| H46C136 | CEA | $IgG_{2a}$ | Directed to domain N of CEA |
| C110 F(ab')$_2$ | CEA | $IgG_1$ | Directed to a proteinaceous epitope in region N of CEA |
| F36/22 | human breast cancer cell line (MCF-7) | $IgG_3$ | Directed to the 20 amino acid tandem repeat of human mammary mucin |
| M85/34 | mucin preparation of human ascites | $IgM_k$ | Directed to N-acetyllactosamine (Will not bind CEA) |

The reagents for the human anti-mouse antibody assay are chosen such that the antibody components of the capture and indicator reagents will bind human anti-mouse antibodies but will not simultaneously bind to an antigen of interest, such as a CEA molecule, which may be in the test sample. Because the assay reagents will not immobilize and label CEA in the test sample, a positive assay result occurs only if human anti-mouse antibodies are present in the test sample. For example, an indicator reagent was made by labeling the C110 antibody, and different capture reagents were made using the H8C2, C110 F(ab')$_2$, B72.3 and H46C136 antibodies as described in Table 1. Based upon the mouse monoclonal antibody characterizations listed in Table 1, only the assay reagent combination of the labeled C110 antibody and the H8C2 capture antibody would be expected to simultaneously bind and label a CEA molecule because the C110, C110 F(ab')$_2$ and H46C136 antibodies all bind to the same epitope of CEA and therefore cannot simultaneously bind the CEA molecule, and B72.3 does not bind CEA. Therefore, if any of the latter reagent combinations produced a detectable signal, the positive result would be due to the bridging of the assay reagents by human anti-mouse antibodies which can simultaneously bind the immobilized and labeled reagents because those reagents were mouse monoclonal antibodies. It will be appreciated by one skilled-in-the-art, that while the description of this aspect of the present invention focuses upon CEA, the inventive concepts can be advantageously applied to detecting human anti-mouse antibodies in test samples containing other typical tumor-associated antigens.

The positive test result occurs when human anti-mouse antibodies are present in the test sample and react with both the immobilized and labeled mouse monoclonal antibodies, thereby bridging the assay reagents. It should be noted that in this assay configuration the immobilized and labeled antibodies bind to human anti-mouse antibodies because the assay reagents are in fact recognized as antigens by the human anti-mouse antibodies. The bridging reaction occurs independently of the specificities of the immobilized or labeled antibodies, an occurrence which distinguishes the human anti-mouse antibody assay from conventional sandwich immunoassays.

## EXAMPLES

The following examples are provided to more fully explain the present invention. The examples are intended to be specific illustrations of the invention and are not intended to be limitative of the invention. It will be appreciated by those skilled-in-the-art that while sandwich assay formats are well-known, the present invention provides a novel use for such assays. In addition, it will be appreciated that while the examples focus upon CEA, the inventive concepts, as described above, can be applied to assays for other analytes of interest as well as to other interfering antibodies.

Example 1

Selection of Human Anti-mouse Antibody Assay Reagents

The following experiment was performed to determine which antibodies were most suitable for use in an assay for the detection of interfering antibodies such as human anti-mouse antibodies.

a. Preparation of Solid Support and Immobilized Antibody

A series of solid support reaction wells (Immulon-II 96 well plate, Dynatech Laboratories, Chantilly, VA) were coated with a mouse monoclonal capture antibody; either H8C2, C110 F(ab')₂, B72.3 (National Institute of Health, NTIS) or H46C136, as described in Table 1 above. One hundred microliters of monoclonal antibody (at a concentration of 2.0 µg/mL in carbonate buffer, pH 9.6) were placed in each well and incubated overnight at 2-8°C. The wells were then overcoated with bovine serum albumin (BSA; 50 mM phosphate buffered saline [PBS], pH 7.4, containing 1% [w/v] BSA) for one hour at 37°C, to complete the preparation of the capture reagent.

b. Test Samples

Sera were diluted 1:10 in PBS containing 5% fetal bovine serum and 0.1% gentamycin sulfate. The sera were then serially diluted to suitable endpoints (1:20 to 10,240), and a final assay volume (100 µL) was placed in each well. The test samples were incubated in the wells for two hours at 37°C. The samples were then aspirated from the wells, and the wells were washed with an Elisa plate washer (Nunc, Denmark).

c. Labeled Antibody

The indicator reagent was an anti-CEA mouse monoclonal antibody (C110) conjugated to an enzyme label (horse-radish peroxidase). The details of the preparation of such indicator reagents and their suitability for use in binding assays are well-known to those skilled-in-the-art. An exemplary conjugation process is described by Nakane, et al., J. Histochemistry and Cytochemistry, 22(12):1084-1091, 1974. Indicator reagents using the other antibodies were made substantially in accordance with this procedure.

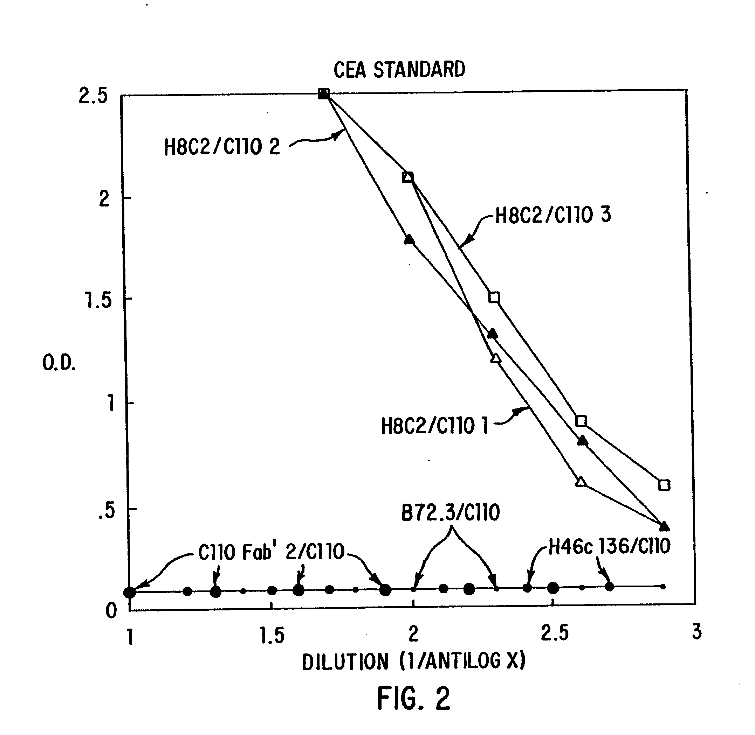

The indicator reagent (100 µL), at an appropriate concentration (e.g., 2.0 µg/mL in PBS containing 5% fetal bovine serum, or 1% bovine serum albumin for the C110 indicator reagent), was added to each well and was incubated in the wells at 37°C for two hours. The wells were then washed with distilled water. A substrate solution (o-Phenylenediamine [OPD]) was added to each well as a signal producing reagent. The enzyme/substrate reaction was developed at room temperature for 30 minutes. The reaction was stopped by the addition of an equal volume of sulfuric acid (I N, 100 µL). The resultant color production was read at 490 nanometers in a plate reader (Biotech Instruments, Winooski, VT). Figure 2 illustrates the results of several sandwich assays in which the different combinations of immobilized capture antibody and labeled antibody were used. The assays were performed substantially in accordance with the above protocol, wherein a serum sample having a known CEA concentration (2415 ng/mL) was used.

The CEA test samples were used in the above experiment to establish that only certain combinations of mouse monoclonal antibodies were appropriate for use as the immobilized and labeled antibody reagents in a CEA sandwich assay format. The capture antibody and labeled antibody must be able to bind different epitopes on the antigen molecule to enable the simultaneous binding of these assay reagents with the antigen.

The results of assays using the above combinations of capture antibody and labeled antibody demonstrated that only the H8C2 capture antibody and the labeled C110 antibody would simultaneously bind CEA, as the H8C2 antibody is specific for the proteinaceous epitope in domain 1 of CEA, while the C110 antibody is specific for a proteinaceous epitope in region N of CEA. The assay using this particular reagent combination was performed on three different days. The combinations of labeled C110 antibody and either immobilized C110 F(ab')₂ or H46C136 capture antibody did not complete the capture antibody/CEA antigen/indicator antibody sandwich complex because these capture antibodies do not bind a substantially different epitope than does the C110 antibody. The B72.3 antibody does not bind any epitope found on CEA, and thus also produced a negative result when used in combination with the CEA-specific C110 monoclonal antibody. Furthermore, the B72.3 antigen is distinct from CEA, and therefore, CEA-specific monoclonal antibodies do not bind to the B72.3 antigen.

Because, the C110/C110 F(ab')₂, C110/B72.3 and C110/H46C136 labeled antibody/capture antibody assay reagent combinations had been shown not to bind CEA, it was concluded that a positive assay result using one of these reagent combinations would indicate the presence of human anti-mouse antibodies, that is, human anti-mouse antibodies would produce a false positive test result due to reagent bridging.

Example 2

Demonstration of Human Anti-Mouse Antibody Titer in the Presence of Antigen

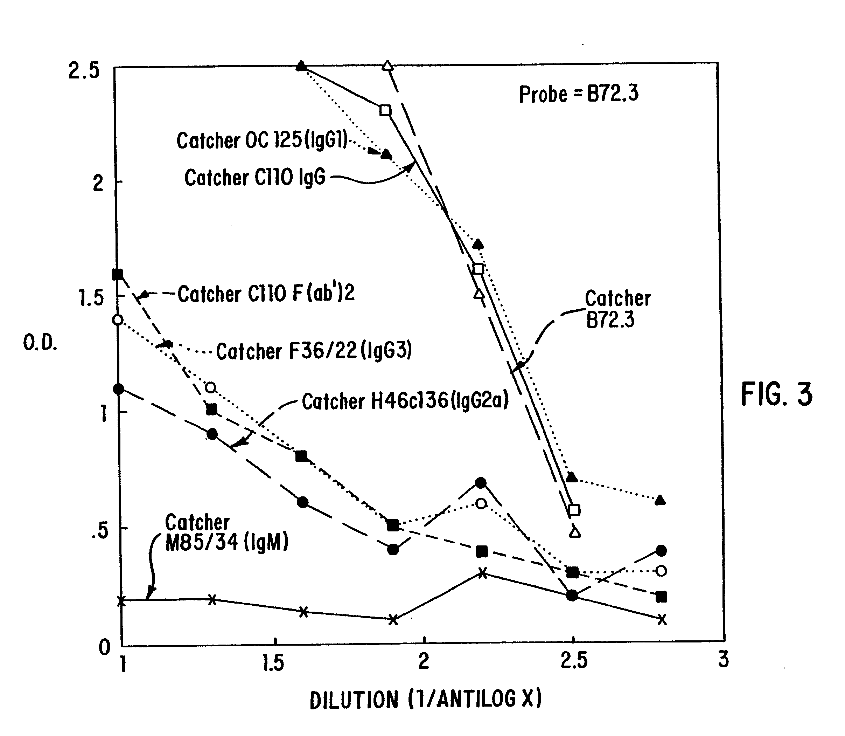

Bridging assays were performed, to detect the presence of human anti-mouse antibodies, using a labeled B72.3 antibody as the probe or indicator reagent and either B72.3, OC125 (Centocor, Malvern, PA), C110, C110 F(ab')₂, F36/22 (Roswell Park Memorial Institute, Buffalo, NY), H46C136 or M85/34 (described in Table I) as the antibody component of the capture reagent. The reagents were prepared, and the assays were performed substantially in accordance with the procedures described in Example 1. The test sample was serum from a patient who had mounted a human anti-mouse antibody response due to previous injections of B72.3 mouse monoclonal antibody chelate in an imaging/therapy protocol (i.e., known presence of anti-isotype human anti-mouse antibodies). The occurrence of labeled B72.3 antibody on the solid support, i.e., a positive test result, indicated that human anti-mouse antibodies were present in the test sample and were bridging the assay reagents. The results of the assays are shown in Figure 3. The only antibody combination shown that would detect antigen (TAG-72, tumor associated glycoprotein antigen) uses the B72.3 antibody as both the capture binding member and the specific binding member component of the indicator reagent. This combination would detect antigen, anti-idiotype human anti-mouse antibodies and anti-isotype human anti-mouse antibodies.

The reagent combinations using a F36/22, H46C136 or C110 F(ab')₂ capture antibody resulted in positive assay results thereby confirming the presence of human anti-mouse antibody in the test sample. While the assay results were positive, the human anti-mouse antibody titer detected with these reagents was about 100-fold less than the human anti-mouse antibody titer measured with the B72.3, OC125 and C110 capture antibody reagents. The assay results demonstrated that the binding to the human anti-mouse antibody was isotype specific: the measured human anti-mouse antibody titer was the same when the capture antibody was one of the three mouse monoclonal antibodies which have different antigenic specificity (e.g., B72.3, OC125 or C110) but the same IgG₁ isotype. When mouse monoclonal antibodies of isotype IgG₂ₐ (e.g., H46C136) or IgG₃ (e.g., F36/22) were used, the apparent human anti-mouse antibody titers were reduced. The decreased reactivity with the C110 F(ab')₂ capture antibody fragment demonstrated that the specificity of human anti-mouse antibodies in this particular patient was directed to the isotypic determinants found in the constant domains of the B72.3 antibody heavy chains.

There was substantially no detectable human anti-mouse antibody titer when the M85/34 antibody (a mouse IgM monoclonal antibody) was used as the immobilized capture antibody. The M85/34 antibody recognizes the I/i blood group antigens (N-acetyllactosamine derivative), and will not bind to CEA, and only rarely does the M85/34 epitope occur on the same molecule together with an epitope recognized by B72.3 or OC125. The use of the mouse IgM antibody in the assay demonstrated the utility of mouse IgM for the detection and measurement of the analyte of interest even in the presence of human anti-mouse antibodies. It illustrated that human anti-mouse antibodies which are directed towards mouse IgG isotype monoclonal antibodies did not strongly cross-react with mouse IgM isotype monoclonal antibodies. Therefore, a mouse antibody can be used as the capture antibody in an assay and human anti-mouse antibody interference can still be avoided, if that mouse antibody has an isotype which is different from the isotype of the interfering antibody.

Example 3

Human Anti-Mouse Antibody Assay to Detect Anti-Idiotype Antibody

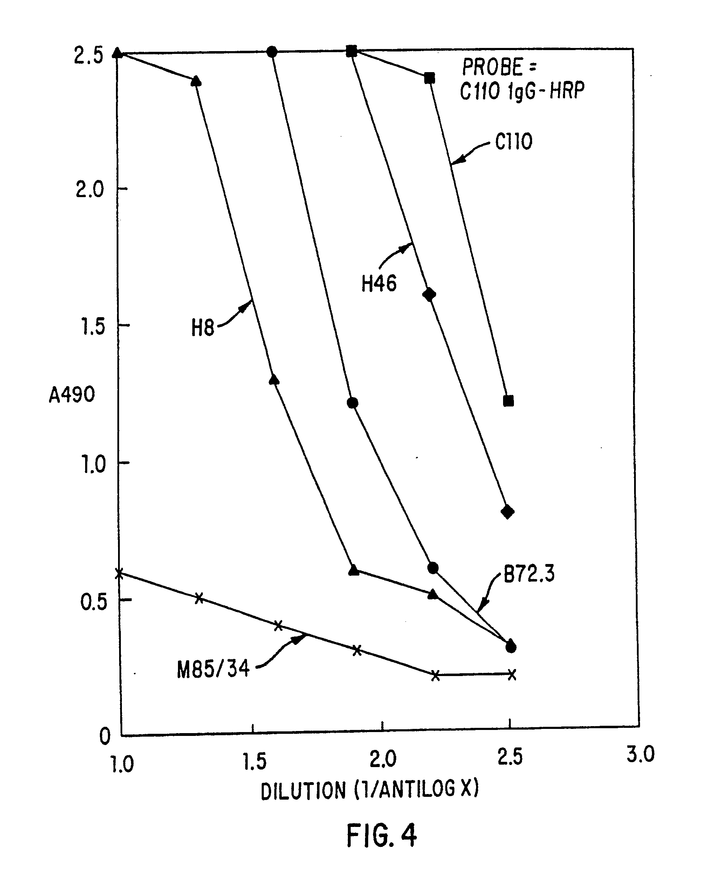

A human anti-mouse antibody bridging assay was designed and used to measure and characterize the immune response of a patient who had been injected with mouse monoclonal antibody C110. The patient's human anti-mouse antibody response resulted in a higher reactivity of his serum in an assay that could only measure human anti-mouse antibodies than in an assay that could measure CEA and that can be interfered with by the presence of human anti-mouse antibodies. The antibody titers observed with the C110 capture reagent/C110 indicator reagent combination were higher than the titers observed with any other monoclonal antibody combination tested and reflected an anti-idiotype specific response. The high reactivity of the C110 indicator reagent/H46C136 capture reagent combination, as shown in Figure 4, indicated that the H46C136 antibody shares one or more idiotypic determinants with C110. This observation is consistent with the CEA-epitope specificity of H46C136, which is similar to that of C110, as described in Table 1 above.

Example 4

Human Anti-Mouse Antibody Assays

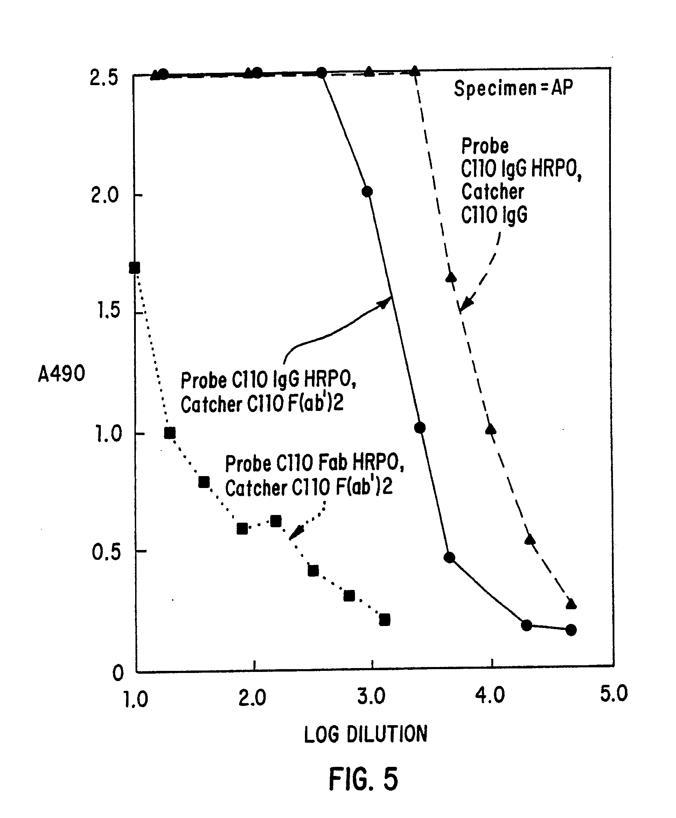

A bridging assay was designed in which a $F(ab')_2$ fragment of the C110 to antibody was used as the immobilized capture antibody in combination with a labeled C110 antibody fragment. The assay reagents were made, and the assay protocol was performed, substantially in accordance with the methods described in Example 1. The test sample was from a patient having a known anti-isotype human anti-mouse antibody response caused by a previous injection of B72.3 mouse monoclonal antibody. This reagent combination was compared to a C110 capture antibody/C110 labeled antibody combination and a C110 capture antibody fragment/C110 labeled antibody combination, as described in Example 3. The label used in each assay was horseradish peroxidase. The assay results, presented in Figure 5, demonstrated that the patient's anti-isotype antibodies were directed substantially towards the heavy chain or constant fragment portion of the immunoglobulin: the human anti-mouse antibody titer which was measured when using the capture antibody fragment was more than 100-fold less than the titer measured when the intact C110 antibody was used as the immobilized capture antibody. These results show that the use of antibody fragments in both the capture and labeled antibody reagents were less efficient than using intact C110 antibody when measuring actual human anti-mouse antibody titer.

Example 5

CEA Assay Comparisons

The following experiments demonstrated that sandwich assays employing a combination of polyclonal antibody and monoclonal antibody reagents measured levels of human anti-mouse antibody better than sandwich assays in which only monoclonal antibodies were used. A plasma sample, from a patient who had been previously injected with mouse monoclonal antibody (B72.3), was treated as described below and was tested using several different commercially available CEA assay kits.

Human anti-mouse antibodies were separated from the test sample by passing the specimens through a chromatography column containing Protein A. Human anti-mouse antibodies, but not CEA, bind to Protein A, and therefore, an assessment of the true CEA and human anti-mouse antibody contributions to an observed CEA test value could be estimated by individually assaying the bound and unbound Protein A fractions. The separation of human anti-mouse antibodies and CEA by column chromatography using Protein A is a method well-known in the art. The purification was performed as follows. A Biorad Econocolumn® (10cm x 9mm, Richmond, CA) was packed with Protein A Sepharose® (5 mL; Pharmacia, Piscataway, NJ). The column was equilibrated by washing with loading buffer (50 mL; 1 M glycine, 3 M Tris, pH 8.8). The test sample (5.0 mL) was diluted with loading buffer (5.0 mL) and was then passed through the column. The column effluent was saved and labeled "non-IgG". This fraction contained true CEA, which has no affinity for the resin. The column was then washed with loading buffer (20 mL). The bound IgG, which included human anti-mouse antibodies, was then eluted from the column with 50 mM phosphate buffer adjusted to pH 3.0 with 1 M citric acid. Protein peaks were detected by their absorbance at 280 nm. The fractions that absorbed light at 280 nm after application of the pH 3.0 buffer were labeled "IgG".

Table 2 summarizes the data obtained by assaying the unfractionated plasma specimen ("Specimen"), the true CEA contribution ("Non-IgG"), and the human anti-mouse antibody contribution ("IgG"). The samples were tested using the following CEA assay kits: the Abbott CEA-EIA Monoclonal One-Step, the Abbott CEA-EIA One-Step and the Abbott CEA-EIA Monoclonal (all from Abbott Laboratories, Abbott Park, Illinois); the CEA-Roche® EIA (Hoffman-LaRoche, Inc., Nutley, New Jersey); and the Tandem®-E CEA (Hybritech, Inc., San Diego, California). The assays were performed according to the protocols described in the appropriate package inserts.

The resultant data demonstrated that false elevations of CEA values were obtained with each of the kits which utilized two monoclonal antibodies in a sandwich format. Hybritech's Tandem®-E CEA, the CEA-Roche® EIA, and the Abbott CEA-EIA Monoclonal One-Step and CEA-EIA Monoclonal products yielded apparent CEA values of from 40.9 ng/mL to greater than 100 ng/mL for the specimen which in fact contained little, if any, CEA. The signal in the assays resulted from IgG, i.e., the presence of human anti-mouse antibodies, and not CEA as is shown by the elevated assay results indicated in the last column of Table 2. The Abbott product which utilizes an immobilized guinea pig polyclonal capture antibody/labeled mouse monoclonal antibody sandwich format (CEA-EIA One-Step) exhibited no significant elevations in apparent CEA values, and was not susceptible to false elevations due to high levels of anti-isotype human anti-mouse antibodies.

## TABLE 2

### Anti-Isotype Human Anti-Mouse Antibody Interference
### in Monoclonal Antibody Sandwich Immunoassays

|  | Apparent CEA (ng/ml) | | |
|---|---|---|---|
| Assay | Specimen | Non-IgG | IgG |
| CEA-EIA One-Step | 3.2 | 0.1 | 0.2 |
| CEA-EIA Monoclonal One-Step | 40.9 | 0 | 4.8 |
| CEA-EIA Monoclonal | >60 | 1.1 | >60 |
| Hybritech Tandem®-E CEA | >100 | 0.3 | 71.9 |
| CEA-Roche® EIA | >50 | 0.9 | >50 |

Example 6

CEA Assays Comparisons

Following Acid-Heat Extraction of Human Anti-Mouse Antibodies

In this experiment, human anti-mouse antibodies were separated from the test specimen of Example 5 by denaturation of the human anti-mouse antibodies during an acid-heat pretreatment of the specimen. The incubation of serum specimens at elevated temperatures and mildly acidic pH, as a method for eliminating protein interferents from test samples in CEA assays, is described in U.S. Patent Numbers 4,180,556 and 4,272,504, which are incorporated by reference herein. Such sample pretreatment was found to denature and precipitate human anti-mouse antibodies while leaving most of the CEA unaffected. A comparison of the CEA values on both extracted and untreated test samples provided an index of the human anti-mouse antibody interference in a given assay. One or more control specimens were also treated to estimate CEA loss during the extraction procedure.

Extraction Protocol: Typically, 0.5 milliliter aliquots of test specimen and three control specimens were diluted with 1.2 milliliter aliquots of 0.2 M sodium acetate (pH 5.0). The diluted specimens were incubated in a water bath at 70-90° C for 15 minutes and centrifuged at 1200 x g for ten minutes to remove denatured protein. The supernatants were then assayed.

Assays and data analysis: Aliquots of extracted and non-extracted test specimen and controls were assayed using the Abbott CEA kits and protocols as described in Example 5 above. The CEA values obtained for the extracted specimens were multiplied by three to correct for the dilution with extraction buffer.

The recovery factor was the mean of the ratios of the observed CEA values for the extracted and non-extracted controls. This factor was typically 1.0 for extractions at 70°C and approximately 0.75 for 90°C incubations. Thus, at 90°C approximately 25% of the CEA was assumed to be denatured.

To evaluate a specimen for human anti-mouse antibody interference, the CEA value for the extracted specimen (corrected for dilution as above) was divided by the recovery factor, and this corrected CEA value was then compared to the CEA value obtained from the non-extracted specimen. An increase in the apparent CEA value following extraction suggested the occurrence of a false negative in the untreated sample, caused by the presence of human anti-mouse antibodies, whereas a decrease in the apparent CEA value following extraction indicated false positive human anti-mouse antibody interference in the assay of untreated sample. The assay results are presented in Table 3.

The data shown in Table 3 demonstrated that the polyclonal capture antibody/monoclonal labeled antibody sandwich assay format (CEA-EIA One-Step) produced equivalent results for both extracted and untreated test samples. In contrast, the double monoclonal assay formats yielded anomalously high results on non-extracted specimens due to the human anti-mouse antibody-mediated bridging of the capture reagent and indicator reagent antibodies.

## TABLE 3

### Comparison of Extracted and Untreated Plasma

| Assay | Untreated | Acid/Heat |
|---|---|---|
| Abbott CEA-EIA One-Step | 2.8 | 3.1 |
| Abbott CEA-EIA Monoclonal One-Step | 56.6 | 3.3 |
| Abbott CEA-EIA Monoclonal | 540 | 15.2 |

Example 7

Human Anti-Mouse Antibody Interference With CEA Assay Results

The following procedure demonstrated the extent to which human anti-mouse antibody interference contributed to apparent CEA levels. In the assay, excess mouse serum was added to the test sample (as used in Example 5) to reduce human anti-mouse antibody-mediated bridging. The addition of normal mouse serum to a specimen containing human anti-mouse antibodies is known in the art to be partially effective in reducing human anti-mouse antibody interference. The excess mouse antibody competes with the labeled and/or immobilized reagent antibodies for binding to the human anti-mouse antibodies in the test sample, thereby reducing the amount of human anti-mouse antibodies which are free to bind the assay reagents. An index of the human anti-mouse antibody interference in a given assay was provided by the comparison of CEA values for test samples in the presence and absence of high concentrations of excess mouse IgG.

Protocol: Typically, 0.5 milliliter aliquots of specimen were diluted with 1.0 milliliter of normal mouse serum. Aliquots of the diluted and undiluted specimens were assayed using the kits and assay protocols as described in Example 5. The CEA values obtained for the diluted specimens were multiplied by three to correct for the dilution with mouse serum. To evaluate a specimen for human anti-mouse antibody interference, the CEA value for the specimen containing mouse serum (corrected for dilution as above) was compared to the CEA value obtained for the untreated specimen. A significant increase in apparent CEA, in the presence of mouse serum would indicate a false negative interference by human anti-mouse antibodies. A decrease in apparent CEA, in the presence of mouse serum would indicate a false positive interference by human anti-mouse antibodies in the assay. The assay results are presented in Table 4.

The data shown in Table 4 demonstrated that the immobilized polyclonal antibody/labeled monoclonal antibody sandwich format (e.g., CEA-EIA One-Step) provided substantially equivalent CEA values in the presence or absence of excess mouse serum. In contrast, each of the remaining double monoclonal sandwich assays yielded much lower CEA values following the use of the excess mouse antisera blocking agent, thereby indicating significant human anti-mouse antibody interference in these assays.

## TABLE 4

### Effect of Mouse Serum on CEA Values

| | Measured CEA Value (ng/mL) | |
|---|---|---|
| Assay | No Mouse Serum | With Mouse Serum |
| Abbott CEA-EIA One-Step | 2.0 | 2.8 |
| Abbott CEA-EIA Monoclonal One-Step | >80 | 2.8 |
| Abbott CEA-EIA Monoclonal | >60 | 5.6 |
| Hybritech Tandem®-E CEA | >100 | 4.6 |
| CEA-Roche® EIA | >50 | 3.7 |

Example 8

Analyte Recovery Assay

The following experiment demonstrated that human anti-mouse antibody-induced false negative and false positive results were avoided when the polyclonal/monoclonal sandwich assay format was used in combination with the addition of mouse serum to the test system. The fact that a CEA immunoassay format provided an appropriate value for the test sample (as used in Example 5) was demonstrated when equivalent results were obtained from human anti-mouse antibody extracted aliquots of the test sample, as was shown in Example 7 above. Relatively few of the human anti-mouse antibody specimens available for study, however, exhibit high CEA levels, and if the endogenous CEA content of the specimen is low, only relatively gross positive human anti-mouse antibody interference will be readily demonstrated by such a procedure. Negative interference and low levels of positive interference were best detected using an analyte recovery protocol.

The recovery protocol tested for the presence cf an interferent that was not detectable in the test sample by standard methods, for example, an interferent that is present in low amounts. Theoretically, if a specimen has an endogenous level of analyte and more analyte is spiked into the specimen, then the measured analyte value should be additive. However, if an interferent is present, even in low amounts, then the measured value will not be additive.

Recovery Protocol: Typically, 20 microliter-aliquots of a specimen (containing 500 to 4000 nanograms of CEA per milliliter) were spiked into 1.0 milliliter aliquots of 1) a specimen containing human anti-mouse antibody, 2) a normal serum control and 3) dilution solutions provided in assay kits to dilute specimens whose CEA values fall above the standard curve. Assays were then performed on spiked and unspiked specimens using commercially available CEA assay kits and reagents. The resultant data were evaluated using the following percent-recovery equation:

$$\% \text{ Recovery} = \frac{(\text{ng/mL spiked serum} - \text{ng/mL endogenous serum})}{\text{ng/mL spiked diluent}} \times 100$$

Acceptable recovery values were typically within the range of about 80% to about 120%. Values above this range suggested a positive interference by human anti-mouse antibodies, i.e., a falsely elevated CEA level. Values below this range indicated a negative interference by human anti-mouse antibodies, i.e., a falsely depressed CEA level. The assay results are presented in Table 5.

## TABLE 5

### Recovery of CEA in Various CEA Assay Formats

| Assay Format | Endogenous CEA (ng/ml) | Endogenous + Spiked CEA (ng/ml) | % Recovery |
|---|---|---|---|
| Abbott CEA-EIA Monoclonal One-Step | 22.4 | 28.8 | 42.3% |
| Abbott CEA-EIA Monoclonal | >60 | >60 | * |
| Hybritech Tandem-E® CEA | >100 | >100 | * |
| CEA-Roche® EIA | >50 | >50 | * |
| Abbott CEA-EIA One-Step | 2.0 | 19.1 | 94.4% |
| performed as a two-step assay with mouse serum in specimen diluent | 2.7 | 20.4 | 115.7% |
| performed as a two-step assay without mouse serum in specimen diluent | 1.2 | 9.2 | 57.6% |

\* unable to determine percent recovery

Three of the double mouse monoclonal sandwich assays (Abbott CEA-EIA Monoclonal, Hybritech Tandem-E® CEA, and CEA-Roche® EIA) showed such gross (i.e., positive) interference by human anti-mouse antibodies that the percent recovery was impossible to determine, due to the endogenous (unspiked) specimen reading above the upper limit of each standard curve. The Abbott CEA-EIA Monoclonal One-Step assay showed both positive and negative interference by human anti-mouse antibodies. The endogenous (unspiked) specimen value was elevated due to positive interference, and only 42.3% of the CEA spiked into the human anti-mouse antibody sample was detected or "recovered", thereby demonstrating negative interference.

The Abbott CEA-EIA One-Step assay is a polyclonal capture antibody/monoclonal labeled antibody sandwich assay. The capture antibody is guinea pig antibody immobilized upon a solid phase. The indicator reagent contains labeled mouse monoclonal antibody and mouse IgG in the form of mouse serum. The specimen is incubated simultaneously with the solid phase and indicator reagent. Mouse IgG is added in the polyclonal/monoclonal one-step assay format to prevent the small degree of crossreactivity or low affinity binding, which can occur between human anti-mouse antibodies and non-mouse antibodies, as well as to prevent the aggregation of labeled mouse monoclonal antibodies which readily bind the human anti-mouse antibodies, as shown in Figure 1(d).

No interference by human anti-mouse antibodies was observed in the Abbott CEA-EIA One-Step assay. The endogenous CEA level was consistent with levels measured after heat extraction, as described in Example 7 above, and 94.4% of the CEA spiked into the specimen was "recovered".

Two alternative assay formats were also evaluated with the human anti-mouse antibody specimen, using reagents available in the Abbott CEA-EIA One-Step assay kit. Both formats used a two-step assay protocol, in which sample was first incubated with a specimen diluent (0 ng/mL standard) and a solid phase coated with polyclonal guinea pig antibody. The solid phase was washed to remove unbound sample, and the solid phase was then incubated with an indicator reagent containing horseradish peroxidase-labeled mouse monoclonal antibody. Mouse IgG, in the form of mouse serum (3% v/v), was added to the specimen diluent in the first two-step assay. In the second assay, no mouse IgG was added to the specimen diluent.

The two-step polyclonal/monoclonal format which did not contain mouse IgG in the specimen diluent showed evidence of human anti-mouse antibody-induced interference. Only 57.6% of the CEA added to the specimen containing human anti-mouse antibodies was detectable in this assay format.

The two-step assay in which mouse IgG was added to the specimen diluent appears to be resistant to positive and negative interference by human anti-mouse antibodies. The endogenous CEA concentration was consistent with values obtained after heat extraction, and the assay exhibited suitable recovery (i.e.,

18

115.7%) of the CEA spiked into the specimen.

The data suggest that, without the addition of mouse IgG to the specimen diluent, specimens containing very high titers of human anti-mouse antibodies can exhibit human anti-mouse antibody-induced interference even in a polyclonal capture antibody/monoclonal labeled antibody two-step assay format. Low affinity binding may occur between the human anti-mouse antibodies in the sample and the non-mouse antibodies on the solid phase. Such non-specific binding can prevent the specific binding of analyte by the polyclonal non-mouse antibodies. This low affinity binding can be blocked by the simultaneous incubation of sample, specimen diluent containing mouse IgG, and the solid phase.

Although the one-step and two-step capture polyclonal antibody/labeled monoclonal antibody assays showed no interference by human anti-mouse antibodies when mouse IgG was incubated simultaneously with the specimen and the solid phase, the two-step configuration is the preferred assay format.

In the one-step assay format, the addition of mouse IgG to samples containing very high titers of human anti-mouse antibodies may be sufficient to prevent the false positive assay results caused by the low affinity binding of human anti-mouse antibodies to the non-mouse antibody and the consequent bridging of indicator reagent and the solid phase. However, it may be insufficient to prevent the false negative assay results caused by the aggregation of the mouse monoclonal antibody indicator reagent.

Therefore, the use of the two-step assay format plus the addition of mouse IgG to the test system is expected to be the best method of preventing both the false negative results and the false positive results which can occur when measuring test samples containing very high titers of human anti-mouse antibodies.

Example 9

Human Anti-Mouse Antibody Assay with Neuraminidase Digestion

The ability to measure anti-idiotype-specific human anti-mouse antibodies is potentially restricted to the use of monoclonal antibodies which recognize only a single epitope per antigen molecule because repetitive epitopes result in a positive if both the capture binding member and indicator reagent binding member have the same specificity. For example, to apply the anti-idiotype assay construct to the human anti-mouse antibody assay, one can destroy the epitopes on the TAG-72 antigen which are recognized by the B72.3 antibody. This is illustrated by the destruction of the B72.3 epitope on the antigen molecule, using neuraminidase (Nanase) digestion, as described in co-owned and copending U.S. Patent Application Serial Number 07/123,439, filed November 20, 1987, which is incorporated by reference herein. Sialic acid does not constitute a determinant found in the idiotypic domains of the immunoglobulin (B72.3) because these domains are peptide in nature.

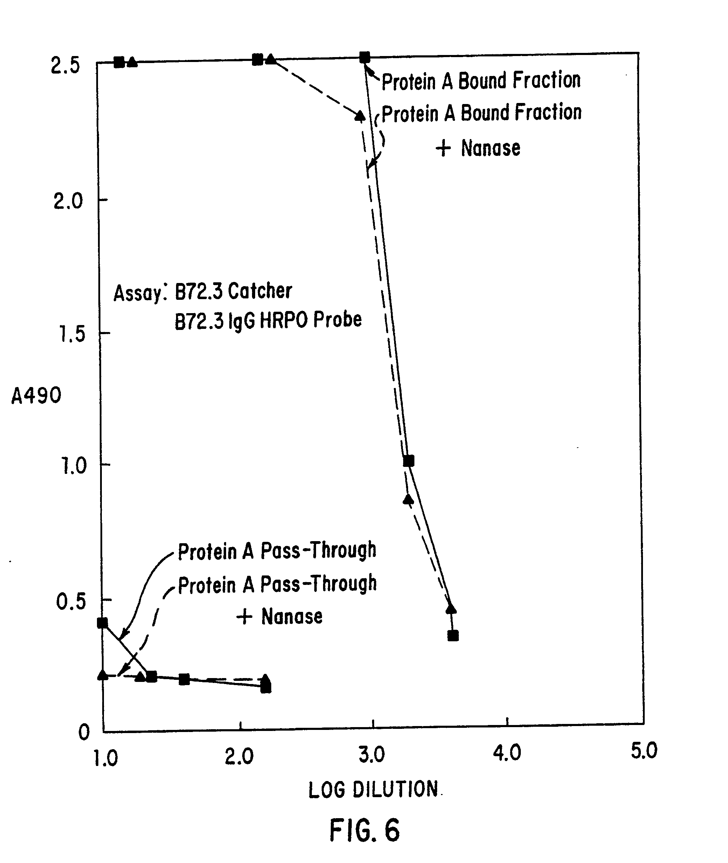

An assay using a B72.3 capture binding member (catcher) and a horseradish peroxidase labeled B72.3 antibody indicator reagent (probe) was performed on a specimen which contained both human anti-mouse antibodies and the TAG-72 antigen. Protein A, an agent which binds the human anti-mouse IgG antibody but not the Tag antigen, was immobilized on Sepharose and used to separate human anti-mouse antibodies from Tag in the test sample. The B72.3 monoclonal antibody requires the presence of sialic acid on the antigen for efficient binding to the antibody. Neuraminidase digestion of the antigen, at 50 mU/mL in phosphate buffered saline for two hours, removes sialic acid from the antigen and thereby reduces antibody reactivity with the antigen, as illustrated in the pass-through results of Figure 6(b) (see Stramignoni, D et al., Int. J. Cancer 31: 543-552, 1983). Figure 6(a) illustrates that neuraminidase digestion of the antigen does not affect the B72.3 antibody's reactivity with the test sample fraction bound to Protein A (i.e., human anti-mouse antibodies).

While the above examples focus on the avoidance of human anti-mouse antibody-induced interference in CEA immunoassays, it will be appreciated by one skilled-in-the-art that the present inventive concepts can be applied to other tumor-associated antigen assays as well as to other assays for analytes of interest. The embodiments described and the alternative embodiments presented herein are intended only to present specific examples of the invention, i.e., the detection of human anti-mouse antibodies and the use of xenogeneic antibodies as reagents in assays which avoid the effects of immune-reaction interferents. Thus, the invention is intended to encompass all equivalents and subject matter within the spirit and scope of the invention as described above and as set forth in the following claims.

## Claims

1. An immunoassay method for determining the presence or amount of an analyte in a patient sample, comprising the steps of:

a) combining the sample with an immunoreactive binding member, thereby forming a mixture, wherein the sample is suspected of containing interferent antibodies produced by the prior immunization of the patient with antibodies from another animal species, wherein said immunoreactive binding member

i ) is xenogeneic in relation to said animal species and does not readily bind to said interferent antibodies, and

i i ) recognizes and binds to the analyte of interest, and wherein the analyte and said immunoreactive binding member form a complex;

b) separating said complex from said mixture; and

c) using a detection means to determine the presence or amount of said complex.

2. The method according to Claim 1, further comprising adding serum containing said immunizing antibodies from said animal species to said mixture containing sample and said first immunoreactive binding member.

3. The method according to Claim 1, wherein said indicator means comprises a detectable label attached to a second immunoreactive binding member, wherein said second immunoreactive binding member binds to said complex thereby forming a detectable labeled complex.

4. The method of claim 2 wherein said animal species is a mouse.

5. A test kit for detecting the presence or amount of an analyte of interest in a patient sample, wherein the sample is suspected of containing human anti-mouse antibodies produced by the prior immunization of the patient with mouse antibodies, comprising:

a) a capture reagent comprising a non-mouse antibody, wherein said antibody

i ) is immobilized upon or is capable of being immobilized upon a solid phase,

i i ) is xenogeneic in relation to the immunizing mouse antibodies and does not readily bind to the human anti-mouse antibodies, and

i i i ) recognizes and binds to the analyte of interest; and

b) an indicator reagent comprising a mouse monoclonal antibody attached to a detectable label, wherein said mouse monoclonal antibody recognizes and binds to the analyte of interest.

6. The kit according to Claim 5, further comprising a sample diluent containing mouse serum.

7. An immunoassay method for detecting the presence or amount of an analyte of interest in a patient sample, comprising the steps of:

a) combining the sample with a first antibody to form a mixture, wherein the sample is suspected of containing human anti-mouse antibodies produced by the prior immunization of the patient with mouse antibodies, wherein said first antibody

i ) is xenogeneic in relation to said immunizing mouse antibodies and does not readily bind to said human anti-mouse antibodies, and

i i ) recognizes and binds to the analyte of interest;

b) adding an indicator reagent comprising a detectable label attached to an analyte analog or analyte, wherein said indicator reagent competes with the analyte for binding to said first antibody;

c) detecting bound or unbound indicator reagent to determine the presence or amount of the analyte of interest in the patient sample.

8. An immunoassay method for detecting the presence or amount of an analyte of interest in a patient sample, comprising the steps of:

a) combining the sample with an indicator reagent comprising a detectable label attached to a first antibody, wherein the sample is suspected of containing human anti-mouse antibodies produced by the prior immunization of the patient with mouse antibodies, wherein said first antibody

i ) is xenogeneic in relation to said immunizing mouse antibodies and does not readily bind to said human anti-mouse antibodies, and

i i ) recognizes and binds to the analyte of interest;

b) adding an analyte analog or analyte which is attached to or is capable of being attached to a solid phase, wherein said analyte analog competes with the analyte for binding to said first antibody, thereby resulting in bound and unbound indicator reagent;

c) detecting either said bound or unbound indicator reagent to determine the presence or amount of the analyte of interest in the patient sample.

9. A bridging assay for the detection of the presence or amount of human anti-mouse antibodies in a test sample, comprising the steps of:

a) contacting the test sample with a mouse monoclonal antibody to form a test mixture, wherein the human anti-mouse antibodies bind to an isotypic epitope on said mouse monoclonal antibody, thereby forming a mouse monoclonal antibody/human anti-mouse antibody complex;

b) separating said complex from said mixture; and

c) using a detection means to determine the presence or amount of said complex, thereby detecting the presence of anti-allotype and anti-isotype antibodies.

10. The assay according to Claim 9, wherein said mouse monoclonal antibody is an IgG class immunoglobulin.

11. A bridging assay for the detection of the presence or amount of human anti-mouse antibodies in a test sample, wherein the antibodies result from the patient's prior immunization with a mouse antibody, comprising the steps of:

a) contacting the test sample with a mouse monoclonal antibody to form a test mixture, wherein said mouse monoclonal antibody was also the immunizing mouse antibody, and wherein the human anti-mouse antibodies bind to said mouse monoclonal antibody, thereby forming a mouse monoclonal antibody/human anti-mouse antibody complex;

b) separating said complex from said mixture; and

c) using a detection means to determine the presence or amount of said complex, thereby detecting the presence of anti-idiotype and anti-isotype antibodies.

12. A bridging assay for the detection of the presence or amount of human anti-mouse antibodies in a test sample, wherein the antibodies result from the patient's prior immunization with a mouse antibody, comprising the steps of:

a) contacting the test sample with a mouse monoclonal antibody fragment to form a test mixture, wherein said fragment is a fragment of the immunizing mouse antibody, and wherein the human anti-mouse antibodies bind to said fragment, thereby forming a mouse monoclonal antibody fragment/human anti-mouse antibody complex;

b) contacting said complex with an indicator reagent comprising a detectable label attached to a second identical antibody fragment, thereby forming a labeled complex; and

c) detecting said labeled complex, thereby detecting the presence of anti-idiotype and anti-isotype antibodies.

HAMA BRIDGING OR COMPETITION

SOLID PHASE ANTIBODY    HUMAN ANTI-MOUSE ANTIBODY    ENZYME-LABELED ANTIBODY FRAGMENT

FIG. 1

ISOTYPE SPECIFIC

HEAVY CHAIN SPECIFIC

FIG. 1(a)

ISOTYPE SPECIFIC

LIGHT CHAIN SPECIFIC

FIG. 1(b)

IDIOTYPE SPECIFIC

**FIG. 1(c)**

IDIOTYPE SPECIFIC

IDIOTYPE SPECIFIC

**FIG. 1(d)**

**FIG. 1(e)**

FIG. 2

FIG. 3

FIG. 4

FIG. 5

FIG. 6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CLIN. CHEM., vol. 34, no. 1, 1988, pages 27-33; L.M. BOSCATO et al.: "Heterophilic antibodies: a problem for all immunoassays" * The whole document, especially page 32, column 2, lines 29-65 * | 1-4,12 | G 01 N 33/53 G 01 N 33/543 |
| X | CLIN. CHEM., vol. 32, no. 8, 1986, pages 1491-1495; L.M. BOSCATO et al.: "Incidence and specificity of interference in two-site immunoassays" * The whole document, especially page 1494, column 2, line 31 - page 1495, column 2, line 21 * | 1-6,12 | |
| Y | | 7-11 | |
| X | EP-A-0 315 447  (TEIJIN LTD) * Column 4, line 37 - column 5, line 7; column 5, line 48 - column 6, line 17; column 8, lines 45-58 * | 1,3,4 | |
| Y | | 9-11 | |
| Y | EP-A-0 177 191  (SERONO DIAGNOSTICS PARTNERS) * Page 3, line 23 - page 5, line 8 * | 8 | TECHNICAL FIELDS SEARCHED (Int. Cl.5) |
| Y | CLIN. CHEM., vol. 34, no. 9, 1988, pages 1843-1846; J. FOLAN et al.: "Solid-phase enzymoimmunoassay of estrone in serum" * Page 1843, column 2, lines 34-48; page 1844, column 1, lines 44-60 * | 7 | G 01 N |

The present search report has been drawn up for all claims

| Place of search | Date of completion of search | Examiner |
|---|---|---|
| The Hague | 10 April 91 | LUZZATTO E.R.P.G.A. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document